# EUROPEAN PATENT SPECIFICATION

(11) **EP 2 185 235 B1**
(45) Date of publication and mention of the grant of the patent: **16.03.2016**
(21) Application number: 08787502.7
(22) Date of filing: 27.08.2008
(51) Int. Cl.: A61N 1/05, A61N 1/36

(54) **DEVICE FOR REDUCING WEIGHT**
VORRICHTUNG ZUR GEWICHTSREDUKTION
DISPOSITIF DE RÉDUCTION DE POIDS

(30) Priority: 28.08.2007 WO PCT/IB2007/002466
(43) Date of publication of application: 19.05.2010
(73) Proprietor: Institut National de la Recherche Agronomique (INRA), 75338 Paris Cedex 07 (FR); Centre Hospitalier Universitaire De Rennes, 35033 Rennes Cedex (FR)
(72) Inventor: BOBILLIER, Eric, 35740 Pace (FR); MALBERT, Charles-Henri, 35170 Bruz (FR); BIRABEN, Arnaud, 35700 Rennes (FR); VAL-LAILLET, David, 35650 Le Rheu (FR)
(74) Representative: Blot, Philippe Robert Emile
(86) International application number: PCT/EP2008/061204
(87) International publication number: WO 2009/027425

(56) References cited:
- WO-A-2004/044947
- US-A- 4 408 608
- US-A- 5 314 457
- US-A1- 2004 267 344
- US-A1- 2006 190 053

## Description

The present description relates to a method for reducing weight through vagus nerve stimulation and to a device for use in such treatment. The invention is set out in the appended claims.

Obesity is one of the largest health problems in the western countries. It may lead to numerous secondary pathology, such as diabetes, sleep apnea, heart diseases, pulmonary diseases; osteoarthritis. The aetiology of obesity is always multifactorial, including genetic and environmental factors. The ideal treatment of obesity is not yet available.

Introduced during the past decade, Vagus Nerve Stimulation (VNS) is commonly used to treat refractory epilepsy (Kramer and Hufnagel "Vagus nerve stimulation: Current status in epilepsy therapy." Aktuelle Neurologie 30:344-349, 2003). Accordingly, devices intended for VNS have received wide approval from regulation authorities of various countries. VNS has an acute effect on epileptic seizures and a progressive prophylactic effect on both severity and frequency of the seizures.

VNS has also been proposed in the frame of obesity treatment. Thus WO 02/062291 describes a method and device for treating obesity in an individual through sub-diaphragmatic vagus nerve stimulation with an electrical pulse signal. In particular, there is described a device for reducing weight in an individual, the device comprising a generator adapted to produce an electrical signal and at least a first set of electrodes comprising two electrodes able to be connected to the generator, the electrodes being intended to be fixed to a first vagus nerve of the individual at a predefined distance one from another, to apply the electrical signal to a portion of the first vagus nerve located between the electrodes.

However, when the generator does not impose an electrical signal to the nerve, a voltage could appear between the electrodes of the same set of electrodes. This voltage is potentially noxious for the individual.

Accordingly, it is an object of the present description to provide a method and device for reducing weight in an individual through vagus nerve stimulation while protecting the individual from the detrimental effects of electrical stimulation. US 2004/0267344 and US 4,408,608 both disclose implantable nerve stimulatous provided with means for short-circuiting the electrodes.

Thus, the present description relates to a device for reducing weight in an individual, the device comprising:
- a generator adapted to produce an electrical signal;
- at least a first set of electrodes comprising two electrodes able to be connected to the generator and being intended to be fixed to a first vagus nerve of the individual at a predefined distance one from another to apply the electrical signal to a portion of the first vagus nerve located between the electrodes;
characterized in that it comprises a short-circuiting switch being adapted to be turned on for short-circuiting the electrodes.

The present description also relates to a device as defined above as a surgical implant for reducing weight in an individual.

The present description also relates to a method for reducing weight in an individual, wherein an effective electrical signal is applied peri-diaphragmatically to a portion of a first vagus nerve and optionally to a portion of a second vagus nerve, wherein the electrical signal is in the form of pulses trains, the pulses having amplitudes increasing from 0.1 milliampere to 2.5 milliampere during a progressive phase (PP) and a constant amplitude equal to 2.5 milliampere during a stabilised phase (SP).

The present description further relates to a method for reducing weight in an individual, wherein an effective electrical signal is applied peri-diaphragmatically to a portion of a first vagus nerve and optionally to a portion of a second vagus nerve by at least one device as defined above.

The present description also relates to the use of a device as defined above, for the manufacture of a surgical implant intended for reducing weight in an individual.

Other features of the device, methods and use, will be apparent from what follows.

As intended herein "reducing weight in an individual" relates to a reduction of the fat tissues of the individual. The individual may or may not suffer from obesity. If the individual suffers from obesity, in particular from morbid obesity, then the method amounts to a method for treating obesity. This expression also encompasses a reduction in weight increase.

As intended herein "obesity" relates to a condition characterized by an excess body weight of an individual with respect to the individual's size, as compared to a normal individual. Preferably, as intended herein, a human individual will be said to suffer from obesity, or to be obese, if his Body Mass Index (BMI = weight (kg) / height² (m²)) is of at least 30 kg/m². Individuals with a BMI of 35-40 of greater will be said to be severely obese or morbidly obese. However, the device, methods and use according to the description can be applied to any individual whose body weight, in particular fat body weight, needs to be reduced, be not considered obese or non-obese. When the human individual to be treated is not obese, or has an obesity which is not associated to any weight-related or obesity-related pathologies, or to any obesity-related disease risk factor, such as individuals with a BMI lower than 35-40, the methods of the description should not be considered as therapeutic methods but merely as non-therapeutic methods or aesthetic methods.

As intended herein "weight-related diseases or obesity-related pathologies" relates to pathologies which aetiologies comprise excess weight or obesity. Such pathologies may notably encompass type 2 diabetes, sleep apnea, heart diseases, pulmonary diseases, or osteoarthritis.

As intended herein, "individual" preferably relates to a mammal, in particular a human.

The expression "vagus nerve" designates the cranial nerve X and its various branches. The "first vagus nerve" and the "second vagus nerve" are selected from the ventral vagus nerve (which innervates in part the stomach, the liver and the proximal duodenum) and the dorsal vagus nerve (which innervates in part the stomach and gets lost in the celiac ganglia).

Where an effective electrical signal is applied to both the first and second vagus nerves, either (i) one device according to the description comprising two sets of electrodes, or (ii) two devices according to the description, each device comprising only one set of electrodes, can be used, the sets of electrodes being each applied to a distinct vagus nerve.

The expression "peri-diaphragmatically" designates a portion of the vagus nerve which is located next to the diaphragm, either supra-diaphragmatically or sub-diaphragmatically. Preferably, this portion is located from 1 cm to 2 cm below diaphragm level for the sub-diaphragm position. This position corresponds to a part of the vagus nerve which can be easily freed from connective tissues. For the supra-diaphragm position, the portion is preferably from 1 cm to 2 cm above the diaphragm. This position corresponds to the part of the vagus nerve located immediately above the Plexus gastricus cranialis.

The expression "effective electric signal" relates to a signal which characteristics (such as intensity, amplitude, pulse duration, frequency) enable a reduction of body weight or of body weight increase.

The surgical methodology for implanting the device according to the description or for vagus nerve stimulation is well known to one of skill in the art and may follow that described e.g. by S.A. Reid ("Surgical technique for implantation of the neurocybernetic prothesis." Epilepsia 31:S38-S39, 1990) for epilepsy treatment. Preferably, the device is implanted in a left subclavian area of the individual.

Other aspects of the description will be apparent from the following description and drawings upon which:
- Figure 1 is a simplified partial front view of a mammal body and of the medical device according to the description;
- Figure 2 is a block diagram of the medical device according to the description;
- Figure 3 is a schematic timing chart illustrating a progressive phase and a stabilised phase generated by the medical device according to the description;
- Figure 4 is a schematic timing chart illustrating the pulses trains transmitted to channels A and B during the stabilised phase;
- Figure 5 is a schematic timing chart illustrating two pulses of a pulse train;.
- Figure 6 represents the evolution of body weight in the sham (black dots) and vagal stimulated (black squares) groups (n=4 per group) of Example 4. The body weight of vagal stimulated animals is significantly different from the sham ones at week 6 and after;
- Figure 7 represents the mean difference in food intake in the sham (dashed boxes) and vagal stimulated (black boxes) animals of Example 4. Except for one week, the daily consumption of stimulated animals is always less than the reference level. The invention is set out in the appended claims. The embodiments, aspects or examples of the present description that do not fall within the scope of said claims are provided for illustrative purposes only and do not form part of the present invention.

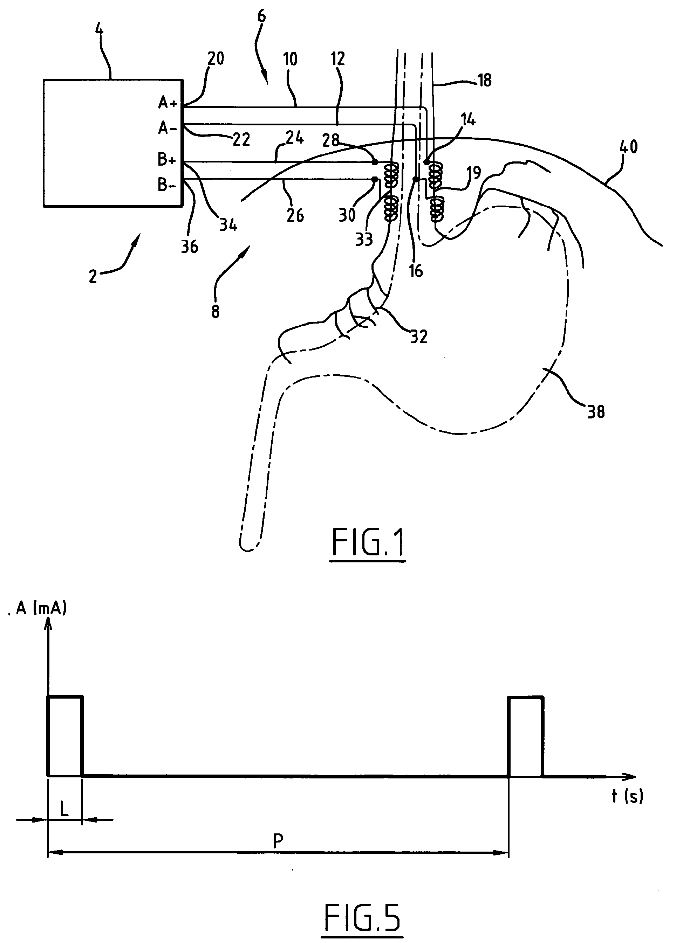

Referring to figure 1, the medical device 2 according to the description is a vagus electro-stimulator device. It comprises an electronic unit 4, a first 6 and second 8 sets of insulated electrodes/leads connected to the electronic unit 4.

The two sets of electrodes 6, 8 are connected to a single electronic unit 4 included in a box. The box is made of titanium. The box is surgically implanted in a subcutaneous pocket of a left subclavian area of the individual.

The first set 6 comprises a first 10 and a second 12 electrodes having distal ends respectively 14 and 16. The distal ends 14 and 16 are intended to be fixed to the dorsal vagus nerve 18 at a predefined distance one from another to apply a current signal to a portion 19 of the dorsal vagus nerve 18 located between these distal ends.

The proximal ends of the first set 6 are linked to output connectors 20, 22 of the electronic unit 4.

The second set 8 comprises a first 24 and a second 26 electrodes having distal ends respectively 28 and 30 intended to be fixed to a ventral vagus nerve 32 at a predefined distance one from another to apply the current signal to a portion 33 of the ventral vagus nerve 32 located between these distal ends.

The proximal ends of the second set 8 are connected to output connectors 34, 36 of the electronic unit 4.

The distal ends 14, 16, 28, 30 of the electrodes are positioned in an area between the top part of the stomach 38 and the lower part of the diaphragm 40.

The electrodes 10, 12, 24, 26 are placed surgically using minimal invasive procedure for the nerves shield surrounding the dorsal and the ventral vagus nerves either immediately before or after the diaphragm 40.

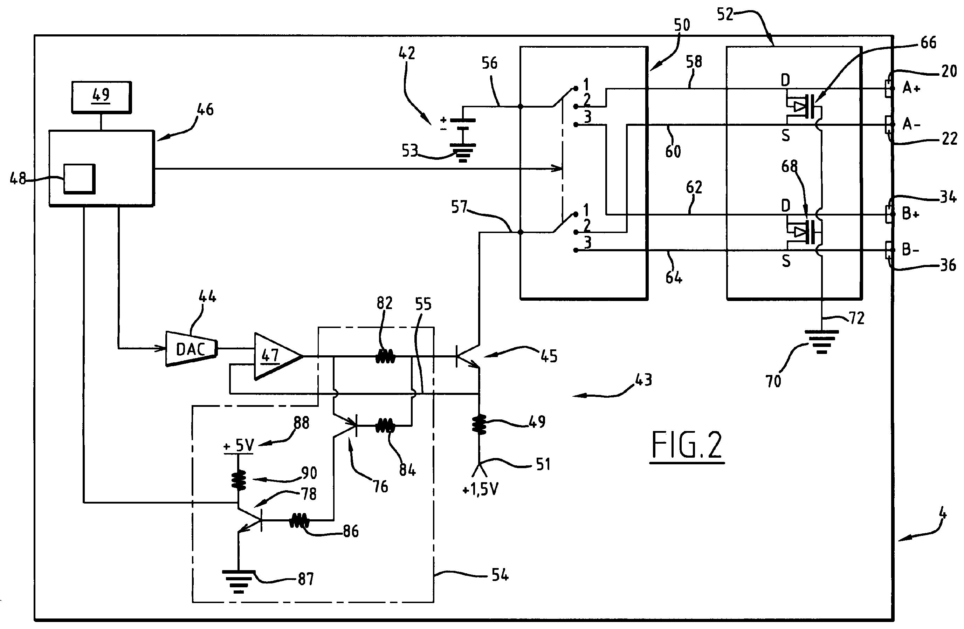

Referring to figure 2, the electronic unit 4 comprises a voltage generator 42, a voltage current converter 43, a digital-to-analogue converter 44 and a microprocessor 46 connected to the digital-to-analogue converter 44.

The voltage generator 42 is a rechargeable battery.

The voltage current converter 43 comprises a transistor 45 adapted to be linked to the voltage generator 42, an amplifier 47 connected to the transistor 45 and a resistance 49 connected to the transistor 45.

The transistor 45 is an n-channel Bipolar Junction Transistor (BJT). It has a base connected to the output of the amplifier 47 via a resistance 82, an emitter linked to the resistance 49, and a collector adapted to be connected to one terminal of the voltage generator 42, the other terminal of the voltage generator being connected to a ground 53.

The amplifier 47 has a negative input connected by a line 55 to the resistance 49 and to the emitter of the transistor 45, and a positive input linked to the digital-to-analogue converter 44.

The voltage current converter 43 comprises a terminal 51 linked to the resistance 49. The terminal 51 is set at a predetermined positive voltage of 1.5 V.

The voltage current converter 43 operates as well known in the state of the art.

The voltage generator 42 and the voltage current converter form a current generator hereafter referenced 42, 43.

The current generator 42, 43 is adapted to apply a current signal to the portion 19 of the dorsal vagus nerve 18 and to the portion 33 of the vagus nerve 32.

The portions of the vagus nerves 19, 33 located between the distal ends 14, 16 and 28, 30 constitute a load for the current generator 42, 43. This load can vary along the time. The current generator 42, 43 is able to adapt the voltage applied to the portions 19 and 33 of the vagus nerves (i.e. load) such as to maintain the amplitude of the current signal approximately constant between the distal ends 14, 16 of the first set and between the distal ends 28, 30 of the second set.

The digital-to-analogue converter 44 is adapted to convert the digital commands of the microcontroller 46 into analogue ones, as well known.

The microprocessor 46 comprises a memory 48 which stores a computer programme defining the current supply commands to the current generator 42, 43.

The microprocessor 46 is able to transmit these commands to the amplifier 47 via the digital-to-analogue converter 44.

The electronic unit 4 also comprises an RF emitter/receiver 49 to send and receive data to and from a handheld programming device, e.g. to amend the computer programme stored in the memory 48.

The electronic unit 4 further comprises a channel selector switch 50 connected to the voltage generator 42, to the terminal 51 and to the microprocessor 46; a short-circuiting switch 52 linked to the channel selector switch 50 and to the output connectors 20, 22, 34, 36 ; and a no compliance detector 54 connected to the microprocessor 46, to the voltage generator 42 and to the terminal 51.

The channel selector switch 50 comprises an input 56 for receiving the current signal of the current generator 42, 43, an output 57 connected to the collector of the transistor 45, two lines 58, 60 connected to the output connectors 20 and 22 for current supplying the dorsal vagus nerve 18, and two lines 62, 64 connected to the output connectors 34 and 36 for current supplying the ventral vagus nerve 32.

The channel selector switch 50 further comprises two resistances having high impedance and not illustrated in figure 2.

The channel selector switch 50 is adapted to connect alternatively the input 56 and the output 57 to the lines 58, 60, to the lines 62, 64 and to the resistances according to the commands received from the microcontroller 46.

In figure 2, the channel selector switch 50 has two contacts not illustrated which are able to select three different electrical terminals noted 1, 2 and 3.

When the contacts of the selector switch 50 are placed on the terminals noted 1, the current generator 42, 43 is linked to the resistances. This corresponds to a high impedance connection.

When the contacts of selector switch 50 are placed on the terminals noted 2, the current generator 42, 43 is linked to the lines 58 and 60. The electrodes 10 and 12 supply a current signal to the portion 19 of the dorsal vagus nerve.

When the contacts of the selector switch 50 are placed on the terminals noted 3, the current generator 42, 43 is linked to the lines 62 and 64. The electrodes 24 and 26 supply a current signal to the portion 33 of the ventral vagus nerve.

The microcontroller 46 is adapted to control the channel selector switch 50 such that the current signal is supplied to the electrodes 10, 12 and the electrodes 24, 26 only during specific periods as described hereafter.

The short-circuit switch 52 comprises a first n channel MOSFET transistor 66 in depletion mode, a second n channel MOSFET transistor 68 in depletion mode.

The first MOSFET transistor 66 has a gate D connected to the output line 58, a source S connected to the output line 60 and a gate connected to a ground 70 through a line 72.

The second MOSFET transistor 68 has a gate D connected to the output line 62, a source S connected to the output line 64 and a gate connected to the ground 70 through the line 72.

Each transistor 66, 68 is configured so that when the potential difference (voltage) between its gate and its source is lower than a predefined threshold, the transistor 66, 68 is turned on, namely the transistor 66, 68 is in a passing current status. The two electrodes 10, 12 (or the electrodes 24, 26) are shortcircuited.

When the potential difference between its gate and its source is greater than this predefined threshold, the transistor 66, 68 is turned off. There is an open circuit between the two electrodes 10, 12 (or the electrodes 24, 26).

The no compliance detector 54 comprises a first 76 and a second 78 transistors. These transistors are n-channel Bipolar Junction Transistors (BJT).

The first transistor 76 is a pnp transistor. It comprises an emitter connected to the output of the amplifier 47 and to the base of the transistor 45 via the resistance 82, a base linked to the base of the transistor 45 via a resistance 84, and a collector connected to the base of the second transistor 78 through a resistance 86.

The second transistor 78 comprises a base, an emitter connected to the ground 87, and a collector connected to the microprocessor 46 and to a voltage source 88 through a resistance 90.

When the electronic unit 4 is operating under non compliance conditions, for instance when an abnormal high impedance appears between the two electrodes 10, 12 (or electrodes 24, 26), the current signal traversing the portions 19 and 33 of the vagus nerves decreases. To maintain the value of the amplitude of the current signal, the current generator 42, 43, via the transistor 45, attempts to compensate this insufficiency by increasing the current signal circulating from the base to the collector of the transistor 45. As a result, the voltage between the terminals of the resistance 82 increases and the current signal passing through the collector and the emitter of the first transistor 76 increases. The current signal delivered by the base of the second transistor 78 increases causing a current signal to be transmitted from the voltage source 88 to the ground 87.

The microcontroller 46 is adapted to detect the appearance of this current signal and to generate an alarm. This alarm reflects the fact that the electronic unit 4 is operating under non compliance conditions i.e a current signal having a abnormal elevated amplitude is delivered to the vagus nerves.

The alarm can also be generated when one of the electrodes 8, 10 , 24, 26 is cut.

The present description also concerns a method for reducing weight wherein an effective current signal is delivered to the vagus nerves along time according to a predefined method which is implemented in the computer program stored in the memory 48.

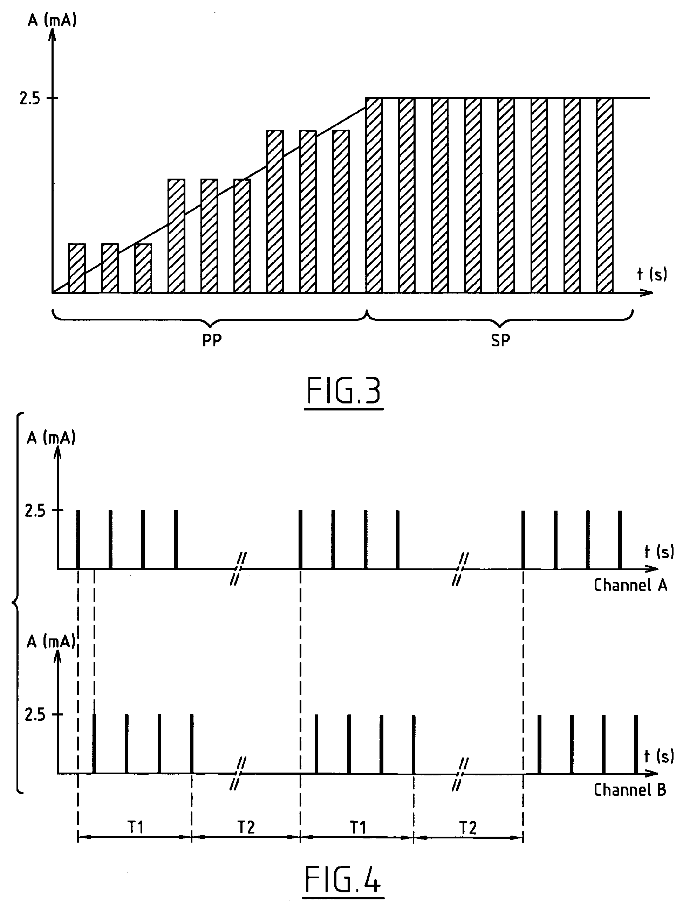

According to this method, the amplitude of the current signal applied to the mammal body increases gradually during an initial phase, hereafter named progressive phase PP, and is maintained constant during a permanent phase, hereafter named stabilised phase SP, illustrated in figure 3.

The progressive phase PP lasts between 10 and 20 days during which the amplitude increases from 0.1 milliampere to 2.5 milliampere.

Preferentially, the progressive phase PP lasts 15 days during which the amplitude increases from 0.1 milliampere to 2.5 milliampere so that the amplitude of the current signal increases each day from 0.16 milliampere.

The stabilised phase SP is preferably maintained until treatment of the individual interrupted. The amplitude of the current signal is maintained equal to 2.5 milliampere during the stabilised phase SP.

The progressive phase PP and the stabilised phase SP comprise emission periods T1 which last each 30 seconds and non emission periods T2 which last each 5 minutes.

During the emission periods T1, the contacts of the selector switch 50 switch from terminal 2 to terminal 3 at a frequency of 60 Hz. The current signal generator 42, 43 provides current signal alternatively to the electrodes 10, 12 (channel A in Figure 4) and to the electrodes 24, 26 (channel B in Figure 4).

When the contacts of the selector switch 50 switch are connected to terminal 2 or to terminal 3, the gate of transistor 66, 68 is linked to the voltage generator 42 and the source of this transistor is linked the terminal 51, the potential difference between the gate and the source of this transistor is greater than the predetermined threshold. The transistor 66, 68 is turned off. The electrodes 10 and 12 are not short- circuited.

The current generator 42, 43 connected to a single battery provides current to both channel A via the first set of electrodes 6 and to channel B via the second set of electrodes 8. Consequently, a pulse is never simultaneously delivered to both vagus nerves, as schematically illustrated with dotted lines in figure 4.

Thus, according to the description, the dorsal 18 and the ventral 32 vagus nerves are never stimulated simultaneously but one after another such that no linkage current appears between the portion 19 of the dorsal vagus nerve 18 and the portion 33 of the ventral vagus nerve 32.

These linkage current signals are damageable. They appear when the impedance of the portion 19 of the dorsal vagus nerve is different from the impedance of the portion 33 of ventral vagus nerve. In this case, a potential difference appears between the portion 19 of the dorsal vagus nerves and the portion 33 of the ventral vagus resulting in the emergence of a current between the dorsal vagus nerve and the ventral vagus nerve.

The current signal generated during the emission period T1 is formed of pulses trains, as schematically illustrated in figure 4. An enlarge view of two consecutive pulses is illustrated in figure 5. The duration L of each pulse is equal to 1 millisecond. The duty cycle of one period of the current signal is equal to 1/30. The period P defined between two consecutive pulses is equal to 33 milliseconds.

During the non emission periods T2, the contacts of the selector switch 50 are connected to the terminal 1. No current signal is provided to the electrodes 10, 12 or to the electrodes 24, 26.

When the contacts of the selector switch 50 switch are connected to terminal 1, no voltage is applied to the gate of the transistor 66, 68. The transistor 66, 68 are turned on. The electrodes 10,12 as well as the electrodes 24, 26 are short circuited.

Advantageously, the device according to the description avoids the appearance of linkage currents between nerves connected to a stimulation device, when the device does not stimulate these nerves.

The described embodiment of the invention needs less energy because the non emission periods T2 are longer than the emission periods T1.

Alternatively, classical MOSFET transistor can also be used in the short-circuit switch 52.

Alternatively, the device can be implemented with a voltage generator instead of the current generator 42, 43. In this case, a voltage signal is applied to the vagus nerves.

Preferably, the device as defined above is a single-use device. A single-use device is a device which can only be implanted once, i.e. the device can not be implanted in a second individual after it has been implanted in a first individual. Indeed, surgical implants are generally only proper for a single use because they may have been damaged or worn during their implantation period in the first individual (i.e. the surgical implants have been consumed by their use) or because they have been in contact with tissues or fluids of the first individual, which renders their use in a second individual hazardous due to possible contaminations.

The present description also contemplates the following non-limitative examples.

### EXAMPLES

### EXAMPLE 1: Efficacy of the device and method for reducing weight

### Material and methods

Two groups of pigs were compared, one group of 6 pigs with Vagal Nerve Stimulation (VNS group) and one group of 6 pigs without VNS, having the same surgical procedure (Sham group). The pigs were large white females about 3 months old and 35 kg weigh at the beginning of the experiment. Surgery was performed under general anaesthesia, after a few days of acclimatization of the animals to their cage. To avoid stimulation of the afferent and efferent vagus nerve fibres joining the larynx, the lungs and the heart, the electrodes were implanted immediately above the diaphragm. The stimulation was applied bilaterally on the ventral and dorsal vagus nerves. Electrodes implantation with bipolar leads required a thoracotomy with the ablation of the left 8^{th} cost; both of the bipolar VNS leads (Cyberonics) were coupled with a device according to the description.

After surgery, each animal had a week for recovering, followed by a week of measures without stimulation. Stimulation was then started in the stimulated group. The parameters of stimulation with the exception of output current intensity were kept unchanged during the entire duration of the experiment (frequency of pulses within the trains, 30 Hz; pulse duration, 1 ms; duration of the train, 30 seconds; and frequency of the trains, 300 s). These values are similar to those usually used in human for epilepsy treatment. During the progressive phase, which lasted 2 weeks, the output current intensity was increased gradually starting at 0.25 mA and ending at 2.5 mA, so that the amplitude increased of 0.16 mA each day.

Once the stabilised phase of stimulation was reached, the animals were fed once every day at the same time and in the same conditions. Access to food was *ad libitum* for half an hour; the quantity of food (2.2 kg) provided was more than the maximal usual ration for pigs of this age and size. The changes in the weight of the pigs were measured as well as other parameters measured on line during the meal, using a weight gauge located under the animal trough: amounts of food consumed at 10 minutes, 20 minutes and 30 minutes and the ingestion speed.

### Results

No significant surgical complications were observed in the course of the experiment.

As expected, no statistical difference was noted in the ingestion parameters between the VNS and Sham groups before VNS was started.

After 3 weeks of stabilized phase stimulation, changes in food intake were significant. Total food intake was reduced at 10, 20 and 30 minutes after the onset of the meal, mainly by reducing the ingestion speed rather than by inducing longer pauses or more frequent pauses during the meal. Results are summarized in **Table 1.**

**Table 1 : Differences in total amount ingested during a meal and in ingestion speed between the sham and VNS groups. Ingested denotes the amount of meal ingested 10, 20 and 30 minutes after the onset of the meal. * denotes a significant difference between groups at p<0.05 (one way ANOVA) Data are mean ± SE.**

| | Sham group | VNS group |
|---|---|---|
| Ingested 10 min (g) | 523 ± 23·0 | 391 ± 21.5* |
| Ingested 20 min (g) | 957 ± 26.6 | 765 ± 15.1 * |
| Ingested 30 min (g) | 1508 ± 28.7 | 1123 ± 20.0 * |
| Ingestion speed 10 min (g.min⁻¹) | 56 ± 3.1 | 40 ± 0.5 * |
| Ingestion speed 20 min (g.min⁻¹) | 50 ± 3.3 | 40 ± 1.9 * |
| Ingestion speed 30 min (g.min⁻¹) | 44 ± 5.1 | 37 ± 5.6 * |

The weight of the pigs was the same before VNS in both groups (38,9 kg on average for the Sham group *vs*. 39,07 kg on average for the VNS group). However, weight increase was significantly reduced post-stimulation in the VNS group with respect to the Sham group (53,75 kg on average for Sham group vs. 46,8 kg on average for the VNS group; p=0,21 using a non-parametric Krustal-Wallis test). Thus, the average weight gain per pig was of 14,85 kg for the Sham group and 7,72 kg for the VNS group.

### EXAMPLE 2: Efficacy of the device and method for altering food preference.

### Material and methods

Two groups of pigs were compared, one group of 4 pigs with Vagal Nerve Stimulation (VNS group) and one group of 4 pigs without VNS, having the same surgical procedure. These animals were different from those used for **Example** 1. However, the surgical preparation and vagal nerve stimulation procedure were identical to that previously described

The animals were feed three times daily (9H30, 12H00 and 16H30) for 30 minutes each. At the preset time of the meal, the animal has access to three different meals presented in three troughs randomly selected by a dedicated robotic device. This device supplies exactly 600 g of food in each trough so that for each individual meal the animal has the possibility to eat up to 1800 g. The meals were different in their composition: one being identical to standard pig food (Control), the other being enriched in sucrose and the last one being enriched with animal and vegetal fat. The composition of each meal is summarized in **Table 2.**

**Table 2. Composition of the three test meals and energy for 100 g of food.**

| | Control | High Sucrose | High Fat |
|---|---|---|---|
| Proteins (g) | 18.2 | 14.7 | 14.8 |
| Cellulose (g) | 4.0 | 3.2 | 3.2 |
| Fat (g) | 4.0 | 3.2 | 18.3 |
| Fibers (g) | 14.0 | 11.3 | 11.4 |
| Starch (g) | 36.9 | 29.8 | 31.4 |
| Carbohydrates (g) | 5.0 | 19.1 | 4.1 |
| Energy (kcal) | 332.4 | 342.0 | 342.1 |

Each meal was designed to taste largely different while supplying an almost identical amount of energy. During each meal, the weight of the three troughs was continuously recorded. Data correspond to week 4 and 5 after the onset of the stimulation as described in **Example 1.**

### Results

No significant surgical complications were observed in the course of the experiment.

After stimulation, change in alimentary preferences is obvious. Whereas, sham animals preferred almost exclusively the High sucrose diet, the VNS group select also almost exclusively the High Fat diet. The results are summarized in **Table 3.**

**Table 3. Percentage of each three different diets ingested daily. The amount of food ingested for 9H30, 12H00 and 16H30 meals were added. All data are significantly different at p<0.01 (one way ANOVA). Data are mean ± SE**

| | Control | High Sucrose | High Fat |
|---|---|---|---|
| Sham group | 5.4 ± 1.1 | 86.5 ± 5.6 | 8.2 ± 2.2 |
| VNS group | 13.7 ± 2.3 | 4.4 ± 0.6 | 81.8 ± 1.9 |

This change in alimentary preferences might result in a weight gain for the VNS group. However, since the VNS group reduced also the total amount ingested and because the energy content of the three test meals is identical, we observed a weight difference in the VNS compared to the sham group (52.0 ± 5.6 kg versus 64.1 ± 7 .1 kg for the VNS versus sham group).

### EXAMPLE 3: Absence of gastrointestinal side effect induced by vagal nerve stimulation

Vagal nerve stimulation was effective to reduce food intake. However, this might be the result of a slow down in gastric emptying. This reduced emptying in turn is able to induce an increased sensation of fullness which is known to reduce ingestion. This, if it is true, will be damageable to the proposed device and method because in human's gastric stasis aside from being capable to reduce food intake generates epigastric fullness and pain. Therefore, the inventors aimed at demonstrating that the proposed device and method were able to reduce food intake without altering the normal gastric emptying process.

### Material and Methods

Two groups of pigs were compared, one group of 4 pigs with Vagal Nerve Stimulation (VNS group) and one group of 4 pigs without VNS, having the same surgical procedure. These animals were different from those used for **Examples 1 and 2.** However, the surgical preparation and vagal nerve stimulation procedure were identical to that previously described.

Gastric emptying of liquids and solids was evaluated with scintigraphy imaging which is the recognized "gold standard". Experiment was performed as previously described by Blat *et al.* (Blat et al., "Role of vagal innervation on intragastric distribution and emptying of liquid and semisolid meals in conscious pigs", Neurogastroenterol Motil, 13:73-80, 2001). Emptying of liquids was measured after the ingestion of 500 ml -10% Dextrose labeled with 20 MBq ^{99m}Tc-DTPA. Emptying of solids was evaluated after the ingestion of 350g of porridge labeled with 20 MBq ^{99m}Tc-sulfur colloid.

Data corresponded to weeks 4 and 5 after the onset of the stimulation as described in **Example 1**. Differences between sham and VNS group were determined using one way ANOVA at p<0.05.

### Results

Gastric emptying of liquids and solids were not significantly different in sham versus VNS group indicating that VNS acts on food intake without altering gastric motility function. Results are summarized in **Table 4.**

**Table 4: Half emptying time (in minutes) for liquid and solid meals in sham versus VNS groups. Data are significantly different from Sham and VNS groups (one way ANOVA). Data are mean ± SE.**

| | Liquids Half emptying time (min) | Solids Half emptying time (min) |
|---|---|---|
| Sham group | 34.8 ± 8.8 | 76.9 ± 14.0 |
| VNS group | 29.1 ± 6.7 | 71.1 ± 18.9 |

### EXAMPLE 4: Efficacy of the device and method in adult obese individuals

To further evaluate the capability of the device and method according to the invention to be used in clinical practice in adult obese patients, the inventors have designed another experiment involving (1) adult animals instead of pre-pubertal ones and (2) obese animals instead of lean ones. Furthermore, vagal stimulation and surgery were performed in morbidly obese animals to be as close as possible to the pathological setting of the human patients the device and method of the description can be applied to.

### Experimental protocol

Two groups of four miniature pigs (Gottingen mini-pigs, 41.9 ± 1.3 kg initially, 18 months at the beginning of the experiment) were feed *ad libitum* with a western diet supplying energy in excess by 2.5 times the normal requirement during 4 months. After this period, the animals weighted 66.2 ± 4.8 kg and were in a morbid obese state as indicated by insulin and glycemia concentrations.

Once in a morbid obese situation, four animals were fitted with dual vagal electrodes as indicated in **Example 1** connected to two devices according to the invention placed under the skin. The four remaining animals received a sham surgery and two mock devices of the same size and weight as the workings ones were also placed under the skin.

One week after surgery, the devices were powered on as indicated in **Example 1**. During 15 weeks, the weight and diet consumption of the animals were monitored. The animals received the same western diet *ad libitum* as before during this experimental period.

### Results

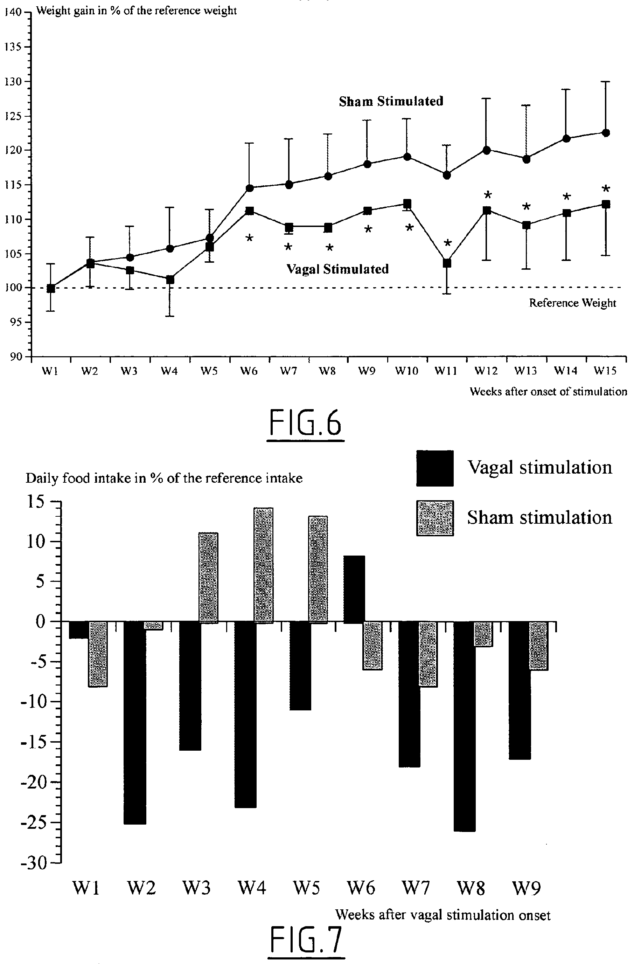

The effect of vagal stimulation are statistically different between the groups 6 weeks after the onset of neurostimulation **(****Figure 6**). The weight difference between the two groups is getting more important along the time course of stimulation. Between weeks 10 and 15, the sham group weights more than their reference weight defined as the weight at week zero (p<0.05). This effect does not vanish during the entire experimental period which lasts 15 weeks.

The food intake of vagal stimulated group is less than control group starting at week 2 after the onset of the neurostimulation (p<0.05, **Figure 7**). This effect does not vanish during the entire experimental period which lasts 15 weeks.

### Conclusions

This experiment demonstrates that Vagal Nerve Stimulation (VNS) is able to reduce the weight of adult obese animals via a significant reduction in food consumption. This reduction takes place without a concomitant change in diet composition or without a reduction in food supply. Finally, the weight reduction is long lasting and without reduction in its potency with time.

The invention is set out in the appended claims that follow:

## Claims

1. An implantable device (2) for reducing weight in an individual, the device (2) comprising:
- a current generator (42, 43) adapted to produce an electrical signal;
- a memory
- at least a first set of electrodes (6) comprising two electrodes (10, 12) adapted to be connected to the generator (42, 43) and configured to be fixed to a dorsal vagus nerve (18) of the individual at a predefined distance one from another to apply the electrical signal to a portion (19) of the dorsal vagus nerve (18) located between the electrodes (10, 12); a short-circuiting switch (52) adapted to be turned on for short-circuiting the electrodes (10, 12), wherein
- the applied electrical signal is an effective current signal which has an amplitude defined by a method implemented in a computer program stored in the memory (48), said amplitude increasing gradually during a progressive phase (PP) and being maintained constant during a stabilised phase (SP)
- the device comprises a second set of electrodes (8) comprising two electrodes (24, 26) adapted to be connected to the generator (42, 43) and configured to be fixed to a ventral vagus nerve (32) of the individual body at a predefined distance one from another to feed with current a portion (33) of the ventral vagus nerve (32) located between the electrodes (24, 26);
- the device comprises a channel selector switch (50) adapted to connect alternatively the first set of electrodes (6) and the second set of electrodes (8) to said generator (42, 43) according to a predefined frequency to transmit the current generated by said generator (42, 43) alternatively to the dorsal (18) and to the ventral (32) vagus nerves,
- each phase comprises an emission (T1) period followed by a non emission period (T2),
- the generator is adapted to produce pulses during each emission period (T1), and not to produce pulses during the non emission periods (T2),
- the short-circuiting switch short-circuits the electrodes of the first and of the second sets during the non emission period (T2), and
- during the emission period (T1), the channel selector switch connects alternatively the first set of electrodes and the second set of electrodes to said generator according to the predefined frequency to transmit the current generated by said generator alternatively to the dorsal and to the ventral vagus nerves.

2. The device (2) according to claim 1, wherein the short-circuiting switch (52) comprises a MOSFET transistor having a gate connected to a ground (70), a gate (D) linked to one electrode (10) and a source (S) connected to the other electrode (12).

3. The device (2) according to claim 2, further comprising a voltage generator (42, 50) adapted to apply a voltage to the source (S) of the short-circuiting switch (52) for controlling its turn on and turn off.

4. The device (2) according to any of claims 1 to 3, wherein the generator (42, 43) is adapted to adjust the voltage applied to at least the portion (19) of the dorsal vagus nerve (18), and wherein the device (2) comprises a detector (54) adapted to generate an alarm when the amplitude of the current is superior to a predefined threshold.

5. The device (2) according to any of claims 1 to 4, wherein the generator (42, 43) is adapted to produce pulses trains, the pulses having amplitudes increasing from 0.1 milliampere to 2.5 milliampere during the progressive phase (PP) and a constant amplitude equal to 2.5 milliampere during the stabilised phase (SP).

6. The device (2) according to claim 5, wherein the progressive phase (PP) is an initial phase lasting between 10 and 20 days.

7. The device (2) according to claim 5 or 6, wherein the pulses trains have a period of 33 milliseconds and the pulse (L) lasts 1 millisecond.

8. The device (2) according to claim 7, wherein the emission period (T1) lasts for 30 seconds and the non emission period (T2) lasts for 5 minutes.

9. The device (2) according to any of claims 1 to 8, wherein the individual is an obese individual.

10. A surgical implant intended for reducing weight in an individual, comprising an implantable device as defined in any of claims 1 to 9.

11. The surgical implant according to claim 10, wherein the individual is obese.

12. The surgical implant according to claim 10 or 11, wherein the individual is morbidly obese.

## Patentansprüche

1. Implantierbare Vorrichtung (2) zur Gewichtsverringerung in einem Individuum, wobei die Vorrichtung (2) folgendes umfasst:
- einen Stromgenerator (42, 43), der geeignet ist, um ein elektrisches Signal zu erzeugen;
- einen Speicher;
- mindestens einen ersten Elektrodensatz (6), der zwei Elektroden (10, 12) umfasst, die mit dem Generator (42, 43) verbunden sind und gestaltet sind, um an einem dorsalen Vagusnerv (18) des Individuums in einem vorbestimmten Abstand zueinander befestigt zu werden, um das elektrische Signal an einem Abschnitt (19) des dorsalen Vagusnervs (18) anzulegen, der zwischen den Elektroden (10, 12) lokalisiert ist;
- einen Kurzschlussschalter (52), der geeignet ist, angeschaltet zu werden, um die Elektroden (10, 12) kurzzuschließen, wobei
- das angelegte elektrische Signal ein wirksames Stromsignal ist, das eine Amplitude hat, die durch eine Methode definiert ist, die in einem Computerprogramm implementiert ist, welches in dem Speicher (48) gespeichert ist, wobei die Amplitude während einer progressiven Phase (PP) graduell zunimmt und während einer stabilisierten Phase (SP) konstant gehalten wird,
- die Vorrichtung einen zweiten Elektrodensatz (8) umfasst, der zwei Elektroden (24, 26) umfasst, die geeignet sind, um mit dem Generator (42, 43) verbunden zu werden, und gestaltet sind, um an einem ventralen Vagusnerv (32) von dem Körper des Individuums in einem vorbestimmten Abstand zueinander befestigt zu werden, um einem Abschnitt (33) des ventralen Vagusnervs (32), der zwischen den Elektroden (24, 26) lokalisiert ist, mit Strom zu versorgen,
- die Vorrichtung einen Kanalwählschalter (50) umfasst, der geeignet ist, um alternativ den ersten Elektrodensatz (6) und den zweiten Elektrodensatz (8) mit dem Generator (42, 43) entsprechend einer vorbestimmten Häufigkeit zu verbinden, um den Strom, der durch den Generator (42, 43) erzeugt wird, alternativ auf den dorsalen (18) und den ventralen (32) Vagusnerv zu übertragen,
- jede Phase eine Emissionsperiode (T1) umfasst, der eine Nicht-Emissionsperiode (T2) folgt,
- der Generator geeignet ist, um während jeder Emissionsperiode (T1) Impulse zu erzeugen und während den Nicht-Emissionsperioden (T2) keine Impulse zu erzeugen,
- der Kurzschlussschalter die Elektroden von dem ersten und dem zweiten Satz während der Nicht-Emissionsperiode (T2) kurzschließt, und
- während der Emissionsperiode (T1), der Kanalwählschalter alternativ den ersten Elektrodensatz und den zweiten Elektrodensatz mit dem Generator entsprechend der vorbestimmten Häufigkeit verbindet, um den Strom, der durch den Generator erzeugt wird, alternativ auf den dorsalen und den ventralen Vagusnerv zu übertragen.

2. Vorrichtung (2) nach Anspruch 1, wobei der Kurzschlussschalter (52) einen MOSFET-Transistor umfasst, der ein Gate, das mit einer Masse (70) verbunden ist, ein Gate (D), das mit der einen Elektrode (10) verbunden ist, und eine Quelle (S) aufweist, die mit der anderen Elektrode (12) verbunden ist.

3. Vorrichtung (2) nach Anspruch 2, ferner umfassend einen Spannungsgenerator (42, 50), der geeignet ist, um eine Spannung an der Quelle (S) von dem Kurschlussschalter (52) anzulegen, um dessen Anschalten und Ausschalten zu kontrollieren.

4. Vorrichtung (2) nach einem der Ansprüche 1 bis 3, wobei der Generator (42, 43) geeignet ist, um die Spannung einzustellen, die an mindestens einem Abschnitt (19) von dem dorsalen Vagusnerv (18) angelegt ist, und wobei die Vorrichtung (2) einen Detektor (54) umfasst, der geeignet ist, um einen Alarm zu erzeugen, wenn die Amplitude des Storms höher als ein vorbestimmter Grenzwert ist.

5. Vorrichtung (2) nach einem der Ansprüche 1 bis 4, wobei der Generator (42, 43) geeignet ist, um Impulsfolgen zu erzeugen, wobei die Impulse während der progressiven Phase (PP) Amplituden haben, die sich von 0,1 Milliampere auf 2,5 Milliampere erhöhen, und während der stabilisierten Phase (SP) eine konstante Amplitude gleich 2,5 Milliampere haben.

6. Vorrichtung (2) nach Anspruch 5, wobei die progressive Phase (PP) eine anfängliche Phase ist, die zwischen 10 und 20 Tagen dauert.

7. Vorrichtung (2) nach Anspruch 5 oder 6, wobei die Impulsfolgen eine Dauer von 33 Millisekunden haben und der Impuls (L) 1 Millisekunde dauert.

8. Vorrichtung (2) nach Anspruch 7, wobei die die Emissionsperiode (T1) 30 Sekunden und die Nicht-Emissionsperiode (T2) 5 Minuten dauert.

9. Vorrichtung (2) nach einem der Ansprüche 1 bis 8, wobei das Individuum ein fettleibiges Individuum ist.

10. Chirurgisches Implantat, das zur Gewichtverringerung in einem Individuum gedacht ist, umfassend eine implantierbare Vorrichtung nach einer dem Ansprüche 1 bis 9.

11. Chirurgisches Implantat nach Anspruch 10, wobei das Individuum fettleibig ist.

12. Chirurgisches Implantat nach Anspruch 10 oder 11, wobei das Individuum krankhaft fettleibig ist.

## Revendications

1. Dispositif implantable (2) pour réduire le poids d'un individu, le dispositif (2) comprenant :
- un générateur de courant (42, 43) conçu pour produire un signal électrique ;
- une mémoire ;
- au moins un premier ensemble d'électrodes (6) comprenant deux électrodes (10, 12) conçues pour être connectées au générateur (42, 43) et configurées de manière à être fixées à un nerf pneumogastrique dorsal (18) de l'individu à une distance prédéfinie l'une de l'autre pour appliquer le signal électrique à une partie (19) du nerf pneumogastrique dorsal (18) située entre les électrodes (10, 12) ;
- un commutateur de mise en court-circuit (52) conçu pour être fermé de manière à court-circuiter les électrodes (10, 12), dans lequel
- le signal électrique appliqué est un signal de courant efficace qui a une amplitude définie par un procédé mis en oeuvre dans un programme d'ordinateur mémorisé dans la mémoire (48), ladite amplitude augmentant graduellement pendant une phase progressive (PP) et étant maintenue constante pendant une phase stabilisée (SP),
- le dispositif comprend un deuxième ensemble d'électrodes (8) comprenant deux électrodes (24, 26) conçues pour être connectées au générateur (42, 43) et configurées de manière à être fixées à un nerf pneumogastrique ventral (32) du corps de l'individu à une distance prédéfinie l'une de l'autre pour fournir un courant à une partie (33) du nerf pneumogastrique ventral (32) située entre les électrodes (24, 26) ;
- le dispositif comprend un commutateur de sélecteur de canal (50) conçu pour connecter alternativement le premier ensemble d'électrodes (6) et le deuxième ensemble d'électrodes (8) au dit générateur (42, 43) conformément à une fréquence prédéfinie pour transmettre le courant généré par ledit générateur (42, 43) alternativement aux nerfs pneumogastriques dorsal (18) et ventral (32),
- chaque phase comprend une période d'émission (T1) suivie d'une période de non émission (T2),
- le générateur est conçu pour produire des impulsions pendant chaque période d'émission (T1), et pour ne pas produire d'impulsions pendant les périodes de non émission (T2),
- le commutateur de mise en court-circuit court-circuite les électrodes des premier et deuxième ensembles pendant la période de non émission (T2), et
- pendant la période d'émission (T1), le commutateur de sélecteur de canal connecte alternativement le premier ensemble d'électrodes et le deuxième ensemble d'électrodes au dit générateur conformément à la fréquence prédéfinie pour transmettre le courant généré par ledit générateur alternativement aux nerfs pneumogastriques dorsal et ventral.

2. Dispositif (2) selon la revendication 1, dans lequel le commutateur de mise en court-circuit (52) comprend un transistor MOSFET ayant une grille connectée à une masse (70), une grille (D) reliée à une électrode (10) et une source (S) connectée à l'autre électrode (12).

3. Dispositif (2) selon la revendication 2, comprenant en outre un générateur de tension (42, 50) conçu pour appliquer une tension à la source (S) du commutateur de mise en court-circuit (52) pour commander sa fermeture et son ouverture.

4. Dispositif (2) selon l'une quelconque des revendications 1 à 3, dans lequel le générateur (42, 43) est conçu pour ajuster la tension appliquée au moins à la partie (19) du nerf pneumogastrique dorsal (18), et dans lequel le dispositif (2) comprend un détecteur (54) conçu pour générer une alarme lorsque l'amplitude du courant est supérieure à un seuil prédéfini.

5. Dispositif (2) selon l'une quelconque des revendications 1 à 4, dans lequel le générateur (42, 43) est conçu pour produire des trains d'impulsions, les impulsions ayant des amplitudes augmentant de 0,1 milliampère à 2,5 milliampères pendant la phase progressive (PP) et une amplitude constante égale à 2,5 milliampères pendant la phase stabilisée (SP).

6. Dispositif (2) selon la revendication 5, dans lequel la phase progressive (PP) est une phase initiale qui dure entre 10 et 20 jours.

7. Dispositif (2) selon la revendication 5 ou 6, dans lequel les trains d'impulsions ont une période de 33 millisecondes et l'impulsion (L) dure 1 milliseconde.

8. Dispositif (2) selon la revendication 7, dans lequel la période d'émission (T1) dure 30 secondes et la période de non émission (T2) dure 5 minutes.

9. Dispositif (2) selon l'une quelconque des revendications 1 à 8, dans lequel l'individu est un individu obèse.

10. Implant chirurgical destiné à réduire le poids d'un individu, comprenant un dispositif implantable selon l'une quelconque des revendications 1 à 9.

11. Implant chirurgical selon la revendication 10, dans lequel l'individu est obèse.

12. Implant chirurgical selon la revendication 10 ou 11, dans lequel l'individu est morbidement obèse.
